# EUROPÄISCHE PATENTSCHRIFT

(11) **EP 0 277 507 B1**
(45) Veröffentlichungstag und Bekanntmachung des Hinweises auf die Patenterteilung: **13.01.1993**
(21) Anmeldenummer: 88100411.3
(22) Anmeldetag: 14.01.1988
(51) Int. Cl.: A61L 11/00, B09B 5/00, A61L 2/06

(54) **Desinfektionsanlage für kontaminierten Krankenhausmüll**
Disinfection device for contaminated household refuse
Dispositif de désinfection pour ordures ménagères contaminées

(30) Priorität: 14.01.1987 DE 3700852; 30.04.1987 DE 3714375
(43) Veröffentlichungstag der Anmeldung: 10.08.1988
(73) Patentinhaber: Roland, Rolf Emil, D-66557 Illingen (DE)
(72) Erfinder: Roland, Rolf Emil, D-66557 Illingen (DE)
(74) Vertreter: Flaccus, Rolf-Dieter, Dr.

(56) Entgegenhaltungen:
- DE-A- 2 550 142
- DE-U- 8 603 896

## Beschreibung

Die Erfindung betrifft eine Desinfektionsanlage für kontaminierten Krankenhausmüll, bestehend aus einem mit steuerbarem Deckel versehenem senkrechten Aufnahmebehälter (1) zur Aufnahme und zur Zerkleinerung des in Plastiksäcken eingegebenen Mülls durch eine Zerkleinerungsvorrichtung (3, 4), dem ein mit Thermo-Öl beheizbarer, ummantelter Schneckenförderer (7) nachgeordnet ist, aus dem der Müll entnommen wird, wobei der Aufnahmebehälter wärmeisoliert ist und diesem eine Vorrichtung (20 bis 31) zur Erzeugung und Einsprühung von Wasserdampf zugeordnet ist und am Abfüll-Ende des Schneckenförderers (7) dessen Ummantelung mit einem Auslaßkanal (12) für kontaminierte Abluft versehen ist, in dem ein Sauggebläse (15) sowie ein Filter (14) angeordnet sind .

Die Offenlegungsschrift DE - A 255 01 42 beschreibt eine Desinfektionsvorrichtung für Krankenhausmüll, bestehend aus einer Zuführvorrichtung, die einen dampfbeheizbaren Mantel und Dampfinjektoren aufweist, einer beheizbaren Sterilisationsförderschnecke und einer abgekühlten Austragsvorrichtung, wobei die latente Wärme der Abkühlung zur Erwärmung des zu sterilisierenden Gutes ausgenützt werden kann.

Aus der DE - U 86 03 796.6 ist weiterhin eine Vorrichtung zur Desinfektion von krankenhausspezifischen, insbesondere infektiösen Abfällen bekannt, mit einem Schneidwerk und einem Schneckenförderer, gekennzeichnet durch eine unterhalb des Schneidwerks angeordnete, den zerkleinerten, zu desinfizierenden Abfall in den horizontal angeordneten Schneckenförderer einlassende Einlaßschleuse und eine an der der Einlaßschleuse gegenüber liegenden Seite des Schneckenförderers angeordnete Auslaßschleuse, wobei der Schneckenförderer von einem erhitztes Thermal-Öl aufnehmdenden Mantel umgeben wird.

Die vorgenannten Desinfektionsanlagen weisen übereinstimmend den Nachteil auf, daß sie nicht den strengen behördlichen Forderungen zur Reinhaltung der Umgebungsluft entsprechen. So ist es beispielsweise unvermeidlich, daß beim Einfüllvorgang des Mülls aus einem Container die Außenluft mit dem kontaminierten Müll und auch mit kontaminierten Anlagenteilen oder mit kontaminierten Abfalleimern in Berührung kommen kann, wobei insbesondere im Zeitraum zwischen Öffnen und Verschließen eines Aufgabeteils der Anlage Bakterien oder Viren in die Umgebungsluft gelangen und zur Ausbreitung von Krankheiten führen können.

Der Erfindung liegt die Aufgabe zugrunde, mit einfachen Mitteln eine Verseuchung der Umgebungsluft in den Einfüllphasen einer thermischen Desinfektionsanlage der beschriebenen Art zu minimieren.

Die Lösung dieser Aufgabe ist in dem Patentanspruch 1 angegeben. Dadurch, daß die Einfüll-Öffnung des Aufnahmebehälters durch einen umgestülpten Müllcontainer verschließbar ist, dessen Inneres mittels eines Strahlrohres durch Wasserdampf desinfizierbar ist, wird im Zusammenwirken mit der am Aufnahmebehälter der Anlage ohnehin vorgesehenen Vorrichtung zur Erzeugung und zum Einsprühen von Wasserdampf eine Verseuchung der Umgebungsluft in den Einfüllphasen wirksam unterbunden. Sehr vorteilhaft ist hierbei das Strahlrohr schwenkbar angeordnet und kann somit durch den gerichteten Dampfstrahl die Innenwände des Müllcontainers schnell und gründlich durch Hitze und Dampf desinfizieren.

Weiter wird durch den fließenden Dampf, der durch alle mit dem eingegebenen Produkt in Berührung gelangenden Anlagenelemente fließt, eine Sterilisation und Desinfektion aller dieser Teile gewährleistet, sodaß auch in Stillstandszeiten eine Verseuchung der Umgebungsluft vermieden ist. Dadurch, daß entsprechend einer weiteren Ausgestaltung der aus zwei gegenläufig rotierenden Schnecken bestehende Schneckenförderer an beiden Schnecken wechselnde Steigungsmaße aufweist, wobei einem Bereich der einen Schnecke mit kleiner Steigung ein Bereich der anderen Schnecke mit großer Steigung gegenüber liegt, wird erreicht, daß das eingegebene Gut auch noch in dem Schneckenförderer einer abscherenden Behandlung unterworfen wird. Der Doppel-Schneckenförderer erhält damit die Funktion einer zusätzlichen Zerkleinerungsmaschine. Zerkleinertes Gut wird durch die Paarung von Bereichen mit unterschiedlicher Steigung intensiv durchgewalkt, sodaß der Wärmeübergang vom beheizten Schneckenmantel auf das behandelte Gut optimiert wird.

In der Zeichnung sind zwei Ausführungsbeispiele des Erfindungsgegenstandes schematisch dargestellt, und zwar zeigen
(Ab hier weiter in der Beschreibung, Spalte 3 der Offenlegungsschrift 0 277 507, Zeile 43 bis Spalte 7, Zeile 15.)
- Fig. 1: eine stationäre Desinfektionsanlage in Seitenansicht, den senkrechten Aufnahmebehälter im oberen Teil geschnitten, ohne die dazugehörige Stahlkonstruktion,
- Fig. 2: den oberen trichterartigen Teil des Aufnahmebehälters zur Darstellung einer Einzelheit,
- Fig. 3: einen senkrechten Schnitt nach der Linie III-III in Fig. 1, und
- Fig. 4: eine fahrbare Desinfektionsanlage in Seitenansicht.

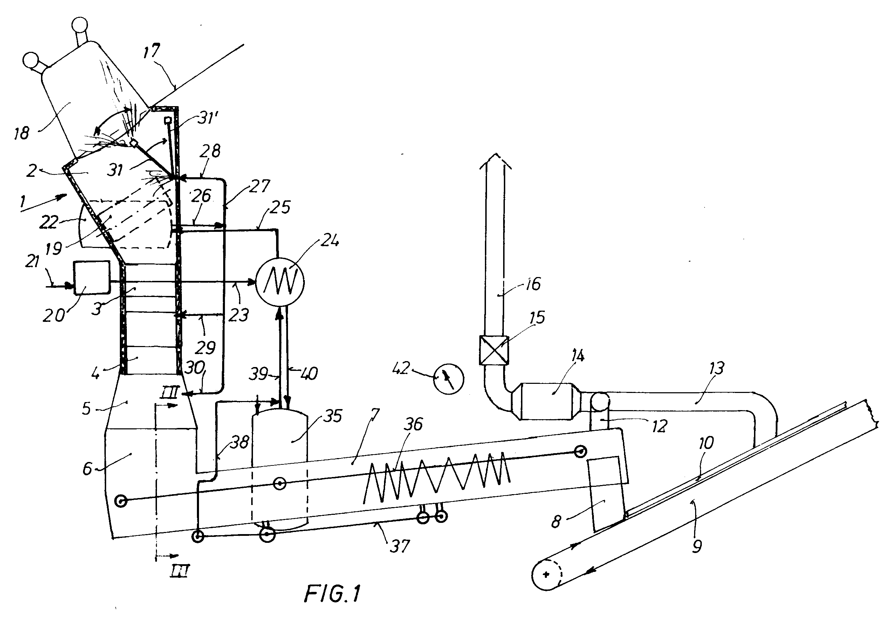

Der senkrechte Aufnahmebehälter 1 nach Fig. 1 besteht aus einem oberen trichterförmigen Teil 2, einem ersten Schneidwerk 3, einem zweiten Schneidwerk 4, einem sich erweiternden Zwischenstück 5 sowie einer Zellenradschleuse 6, welche Teile lediglich durch die ihnen zugeordneten Abschnitte des Aufnahmebehälters 1 dargestellt sind. Die Wandungen des Aufnahmebehälters 1 sind wärmeisoliert. An den unteren Bereich des Aufnahmebehälters unterhalb der zellenradschleuse 6 schließt sich ein mit Thermo-Öl beheizbarer, ummantelter Schneckenförderer 7 an, der aus zwei gegenläufigen Schnecken 7a besteht (Fig. 3). Auch die Ummantelung des Schneckenförderers 7 ist wärmeisoliert.

Am Abfüll-Ende des Schneckenmantels des aufsteigend ausgeführten Schneckenförderers 7 ist ein Fallrohr 8 für desinfizierten Müll vorgesehen, der über einen Bandförderer 9 abtransportiert wird. Das Obertrum des Bandförderers 9 ist durch ein unten offenes Gehäuse 10 abgedeckt, an das sich ein Abluftrohr 13 anschließt. In das Abluftrohr 13 mündet auch ein am Abfüll-Ende des Schneckenförderers vorgesehener Auslaßkanal 12 für kontaminierte Abluft, die zusammen mit der von dem Gehäuse 10 aufgenommenen Abluft einen Aktivkohlefilter 14 durchströmt. An diesen Filter 14 schließt sich der Kamin 16 an, in dem ein Sauggebläse 15 angeordnet ist.

Das obere trichterförmige Teil 2 des Aufnahmebehälters 1 hat eine Öffnung, die durch einen Flachschieber 17 als Deckel verschließbar ist. Die Öffnung des trichterförmigen Teils 2 ist in der Weise ausgelegt, daß ein Müllcontainer 18 in der dargestellten Kipplage, in der die von ihm aufgenommenen Müllsäcke in das Teil 2 hinunterfallen, die Öffnung dicht verschließt. Eine Hubvorrichtung zur Aufnahme und Handhabung des Müllcontainers 18 ist nicht dargestellt.

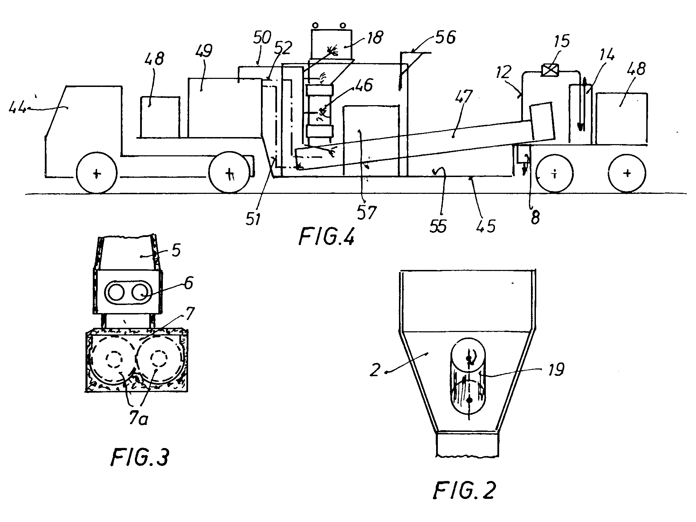

Im trichterförmigen Teil 2 des Aufnahmebehälters 1 ist senkrecht zu einer schrägen Wandung eine exzentrisch gelagerte Walze 19 angeordnet (Fig. 2), die mit einem nicht dargestellten Drehantrieb versehen ist und aufgrund der exzentrischen Lagerung eine taumelnde Bewegung ausführt. Diese rotierende Walze dient dazu, einen Stau durch die aus dem Müllcontainer 18 entleerten Müllsäcke innerhalb des trichterförmigen Teils zu verhindern.

Dem Aufnahmebehälter 1 ist eine Vorrichtung zur Erzeugung und Einsprühung von Wasserdampf zugeordnet, die wie folgt beschrieben wird:

Das beim Betrieb der Anlage über die Leitung 21 zugeführte Wasser wird in einem Wasseraufbereitungsbehälter 20 aufbereitet, um einer Dampferzeugungsanlage 22 zugeführt zu werden. Das über die Rohrleitung 23 geleitete aufbereitete Wasser durchläuft jedoch zunächst den Sekundärkreis eines Wärmeaustauschers 24, aus dem das vorgewärmte Wasser über die Rohrleitung 25 der Dampferzeugungsanlage 22 zugeführt wird. Der hierin erzeugte Dampf wird über die Leitung 26 einer senkrechten Dampfleitung 27 zugeführt, von der drei Abzweige 28, 29 und 30 in das Innere des Aufnahmebehälters 1 führen, um dort in verschiedenen Höhenlagen Dampf einzudüsen. An die Dampf-Zweigleitung 28 für den t trichterförmigen Teil 2 des Aufnahmebehälters 1 ist ein Strahlrohr 31 mit einem rotierenden Dü senkopf bekannter Art schwenkbar angeschlossen, mit dem der entleerte Müllcontainer 18 desinfiziert werden kann, bevor er abgenommen und die Einfüllöffnung des trichterförmigen Teils 2 durch den Flachschieber 17 verschlossen wird. Beim Entleeren eines Müllcontainers 18 ist das Strahlrohr 31 in die Stellung 31' beiseitegeschwenkt, um von den herabfallenden Plastiksäcken nicht beschädigt zu werden.

Das zur Beheizung des Schneckenförderers 7 dienende Thermo-Öl wird in dem Heizkessel 35 so hoch erhitzt, daß in dem Schneckenförderer 7 eine Innentemperatur von ca. 413 K (140 °C) herrscht. Das erhitzte Thermo-Öl wird über beidseitig von dem Schneckenförderer 7 angeordnete Leitungen 36 an mehreren Stellen in die Ummantelung des Schneckenförderers 7 eingeleitet. Das abgekühlte Thermo-Öl wird über die Leitung 37 wahlweise direkt in den Heizkessel 35 rückgeleitet oder über die Leitung 38 einem Steigrohr 39 zugeführt, das an den Primärkreis des Wärmeaustauschers 24 angeschlossen ist. Auf diese Weise wird das dem Dampferzeuger 22 zugeführte Wasser vorgewärmt. Der Primärkreis des Wärmeaustauschers 24 ist ausgangsseitig über die Leitung 40 mit dem Heizkessel 35 verbunden. Durch nicht dargestellte Ventile kann mit oder ohne Inbetriebnahme des Wärmeaustauschers 24 gefahren werden.

Dem Abluftrohr 13 bzw. dem Kamin 16 ist ein Hygrometer 42 zugeordnet, um die Feuchtigkeit der Abluft zu messen und demnach unter Umständen das Einsprühen von Wasserdampf in den Aufnahmebehälter 1 zu steuern.

Die Wirkungsweise der in Fig. 1 beschriebenen stationären Desinfektionsanlage ist folgende:
Wenn die Einfüllöffnung des trichterförmigen Teils 2 durch den Flachschieber 17 geöffnet wird, sorgt eine Folgesteuerung dafür, daß Wasserdampf über die Leitungen 28, 29 und 30 in den Aufnahmebehälter 1 eingesprüht und das Sauggebläse 15 eingeschaltet wird. Hierdurch entsteht im Aufnahmebehälter 1 ein Unterdruck, so daß höchstens Außenluft und Wasserdampf die gesamte Anlage einschließlich des Schneckenförderers 7 durchziehen. Wenn die Einfüllöffnung wie dargestellt durch einen Müllcontainer 18 verschlossen ist, ist die Außenluft abgesperrt. Die herabfallenden Müllsäcke gelangen zuerst in das Schneidwerk 3, nachdem sie durch die rotierende Exzenterwalze 19 beim Herabsinken in Bewegung gehalten sind. Nach der zweiten Zerkleinerung in dem zweiten Schneidwerk 4 fällt der zerkleinerte Müll über die Zellenradschleuse 6 zwischen die gegenläufig rotierenden Schnecken 7a des beheizten Schneckenförderers 7. Hier wird die Desinfektion und Sterilisation des zerkleinerten und bereits mit heißem Wasserdampf behandelten Mülls aufgrund der hohen Innentemperatur vollendet, so daß der desinfizierte Müll über das Fallrohr 8 auf den Bandförderer 9 gelangt und abtransportiert wird. Die kontaminierte Abluft gelangt über die Rohrleitung 12 in den Aktivkohlefilter 14 und dann gereinigt in den Kamin 16. Das flache Gehäuse 10 nimmt allenfalls noch aufsteigende Schwaden des noch erhitzten, desinfizierten Mülls auf,

Es kann vorteilhaft sein, daß die Gänge der beiden Schnecken 7a des Schneckenförderers 7 wechselnde Steigungsmaße aufweisen derart, daß bei der gegenläufigen Rotation der beiden Schnecken ein Bereich der einen Schnecke mit kleiner Steigung einem Bereich der anderen Schnecke mit größerer Steigung gegenüberliegt. Hierdurch wird erreicht, daß das eingegebene Gut auch noch in dem Schneckenförderer einer abscherenden Behandlung unterworfen wird. Der Doppel-Schneckenförderer erhält damit noch die Funktion einer Zerkleinerungsmaschine. Zerkleinertes Gut wird durch die Paarung von Bereichen mit unterschiedlicher Steigung intensiv durchgewalkt, so daß der Wärmeübergang von dem beheizten Thermo-Öl auf das zu behandelnde Gut optimiert wird.

Die in Fig. 4 dargestellte fahrbare Desinfektionsanlage umfaßt eine Zugmaschine 44 sowie einen fahrbaren Unterbau 45 in der Art eines Sattelschlepper-Anhängers bzw. -Aufliegers , auf dem ein Aufnahmebehälter 46 sowie ein beheizbarer Schneckenförderer 47 montiert sind. Der Aufnahmebehälter 46 und der Schneckenförderer 47 entsprechen in verkleinerten Ausführungen den Anlageteilen gemäß Fig. 1. Die Anlage ist in Betrieb, da die Einfüllöffnung des Aufnahmebehälters 46 durch einen umgestülpten Müllcontainer 18 verschlossen ist. Ein Fallrohr 8 am Abfüllende des Schneckenförderers 47 dient auch hier zum Austragen des desinfizierten Gutes. Die kontaminierte Abluft wird über die Abluftrohrleitung 12, das Sauggebläse 15 und den Filter 14 gereinigt abgegeben.

Der Auflieger 45 nimmt ferner die Energieversorgung 48 für Gas sowie Elektrizität auf, um die Heizanlagen sowohl zur Erzeugung des Wasserdampfes als auch zur Beheizung des Thermo-Öls innerhalb der Station 49 zu versorgen. Der erzeugte Wasserdampf wird über die Leitung 50 und verschiedene Abzweige in verschiedenen Höhenlagen des Aufnahmebehälters 46 eingedüst. Die Rohrleitung 51 führt aufgeheiztes Thermo-Öl, wogegen das abgekühlte Thermo-Öl über die Leitung 52 rückgeleitet wird.

Wesentlich ist, daß der Auflieger 45 Stellplätze für eine Mehrzahl von Klein-Containern mit einem Fassungsvermögen von ca. 1 m³ hat. Die Plattform 55 beiderseits des Schneckenförderers 47 ist zur Aufnahme der Klein-Container während des Transportes nach dem Einsammeln vorgesehen. Weitere Stellplätze können mittels einer Brückenkonstruktion oberhalb des Schneckenförderers 47 vorgesehen werden. Zum Auf- und Abladen sowie zum Verbringen von Müllcontainern in Entleer-Stellung im Bereich der Einfüllöffnung des Aufnahmebehälters 46 dient ein heb- und senkbarer Hydraulikkran 56.

Ferner nimmt der Auflieger 45 noch die elektrische Steuerung 57 auf. Im übrigen wird die fahrbare Desinfektionsanlage nach Fig. 4 ebenso betrieben wie die in Fig. 1 dargestellte stationäre Anlage.

## Patentansprüche

1. Desinfektionsanlage für kontaminierten Krankenhausmüll, bestehend aus einem mit steuerbarem Deckel versehenem senkrechten Aufnahmebehälter (1) zur Aufnahme und Zerkleinerung des in Plastiksäcken eingegebenen Mülls, durch eine Zerkleinerungsvorrichtung (3, 4), dem ein mit Thermo-Öl beheizbarer, ummantelter Schneckenförderer nachgeordnet ist, aus dem der Müll entnommen wird, wobei der Aufnahmebehälter wärmeisoliert ist und diesem eine Vorrichtung (20 bis 31) zur Erzeugung und Einsprühung von Wasserdampf zugeordnet ist, und am Abfüll-Ende des Schneckenförderers (7) dessen Ummantelung mit einem Auslasskanal (12) für kontaminierte Abluft versehen ist, in welchem ein Sauggebläse (15) sowie ein Filter (14) angeordnet sind, dadurch gekennzeichnet, daß die Einfüll-Öffnung des Aufnahmebehälters (1) durch einen umgestülpten Müllcontainer (18) verschließbar ist, dessen Inneres mittels eines Strahlrohres (31) durch Wasserdampf desinfizierbar ist.

2. Desinfektionsanlage nach Anspruch 1, dadurch gekennzeichnet, daß das Strahlrohr (31) schwenkbar angeordnet ist.

3. Desinfektionsanlage nach Anspruch 1, mit einem Schneckenförderer, bestehend aus zwei gegenläufig rotierenden Schnecken, dadurch gekennzeichnet, daß die Gänge der beiden Schnecken (7a) wechselnde Steigungsmaße aufweisen, derart, daß bei der gegenläufigen Rotation der beiden Schnecken ein Bereich der einen Schnecke mit kleiner Steigung einem Bereich der anderen Schnecke mit größerer Steigung gegenüber liegt.

4. Desinfektionsanlage nach Anspruch 1, dadurch gekennzeichnet, daß ein Wärmetauscher (24) vorgesehen ist, dessen Primärkreis von dem abgekühlten Thermo-Öl und dessen Sekundärkreis von dem der Dampferzeugungsvorrichtung (22) zugeführten Wasser durchflossen ist.

5. Desinfektionsanlage nach Anspruch 1, dadurch gekennzeichnet, daß von der Steuerung zum Öffnen und Schließen des Deckels (17) eine Steuerung zum Einsprühen des Wasserdampfes in den Aufnahmebehälter ausschließlich bei geöffnetem Deckel abgeleitet ist.

6. Desinfektionsanlage nach Anspruch 1, dadurch gekennzeichnet, daß in dem oberen trichterartigen Teil (2) des Aufnahmebehälters (1) eine waagerechte, rotierende Walze (19) angeordnet ist, die exzentrisch gelagert ist und/oder einen unrunden Querschnitt hat.

7. Desinfektionsanlage nach Anspruch 1, dadurch gekennzeichnet, daß der lichte Querschnitt des Aufnahmebehälters (1) unterhalb der Zerkleinerungsvorrichtung (3, 4) erweitert ist.

8. Desinfektionsanlage nach Anspruch 1 und 6, dadurch gekennzeichnet, daß eine zweiter Zerkleinerungsvorrichtung (4) vorgesehen ist.

9. Desinfektionsanlage nach mindestens einem der Ansprüche 1 bis 7, gekennzeichnet durch einen fahrbaren Unterbau (45) z. B. in der Art eines Sattelschlepper-Anhängers zur Aufnahme der gesamten Desinfektionsanlage einschließlich eines Versorgungsteils (48) zum Mitführen von Heizgas und Wasser für die Dampferzeugung und eines Hydraulikkrans (56) zum Handhaben von Klein-Müllcontainern (18), in dessen Schwenkbereich eine Einrichtung zum Desinfizieren leerer Container angeordnet ist, wobei zumindest beiderseits des beheizten Schneckenförderers (7) Stellplätze (55) für eine Mehrzahl von Klein-Containern mit einem Fassungsvermögen vorgesehen sind, das den Transport von kontaminiertem Gut über Straße erlaubt, und wobei die Gesamthöhe des fahrbaren Unterbaues einschließlich der Anlage die für den Straßenverkehr zulässige Höhe unterschreitet.

10. Desinfektionsanlage nach Anspruch 8, dadurch gekennzeichnet, daß der obere Teil (8) des senkrechten Aufnahmebehälters (1) der Anlage demontierbar oder kippbar ausgeführt ist.

## Claims

1. System for disinfecting contaminated hospital waste, comprising a vertical receptacle (1) having a controllable cover for receiving and comminuting waste contained in plastic bags by means of a comminuting device (3, 4), with a jacketed screw conveyor, which can be heated with thermal oil, being disposed downstream of said receptacle (1) dispensing the waste, wherein said receptacle (1) is provided with a thermal insulation and communicates with a device (20 to 31) for generating and spraying steam, and wherein the jacket of the screw conveyor is, at the dispensing end thereof, provided with an outlet channel (12) for contaminated exhaust air, with an aspirator (15) and a filter (14) being disposed in said channel, characterized in that the feed opening of the receptacle (1) can be closed by means of a waste container (18) turned upside down whereby the interior of the latter can be disinfected with steam by means of a nozzle pipe (31).

2. System in accordance with claim 1, characterized in that the nozzle pipe (31) can be pivoted.

3. System in accordance with claim 1 having a screw conveyor comprising two screws rotating in opposite directions, characterized in that the threads of both screws have different pitches so that when the two screws rotate in opposite senses an area of the one screw with a smaller pitch is opposite an area of the other screw with a larger pitch.

4. System in accordance with claim 1, characterized in that provision is made for a heat exchanger (24) whose primary circuit carries the cooled down thermal oil and whose secondary circuit carries the water supplied to the steam generator (22).

5. System in accordance with claim 1, characterized in that a control unit, which allows steam to be sprayed into the receptacle only when the cover is open, is diverted from the control unit for opening and closing the cover (17).

6. System in accordance with claim 1, characterized in that a horizontal, rotating roller (19), which is eccentric and/or has a non-circular diameter, is disposed in the upper funnel-like part (2) of the receptacle (1).

7. System in accordance with claim 1, characterized in that below the comminuting device (3, 4) the inside diameter of the receptacle (1) is extended.

8. System in accordance with claims 1 and 6, characterized in that provision is made for a second comminuting device (4).

9. System in accordance with at least one of the claims 1 to 7, characterized by a movable support (45), e.g. a trailer as known from trucks or tractor-trailer vehicles, capable of supporting the entire disinfection system including the power supply unit (48) for carrying heating gas and water for generating steam and a derricking crane (56) for handling small waste containers (18), with a device for disinfecting empty containers being provided within the pivoting range of said crane, wherein individual spaces (55) for a plurality of small containers are provided on both sides of the heatable screw conveyor, the volume of said containers allowing transport of the contaminated material across a street, while the total height of the movable support including the system does not exceed the vehicle height allowed in public traffic.

10. System in accordance with claim 8, characterized in that the upper part (8) of the vertical receptacle (1) of the system can be removed or tilted.

## Revendications

1. Installation de désinfection de déchets hospitaliers contaminés, composée d'un réceptacle d'alimentation vertical (1) muni d'un couvercle à commande, servant à recueillir et à déchiqueter les déchets contenus dans des sacs en plastique. Un dispositif de déchiquetage (3, 4) sert à déchiqueter les déchets. Lui fait suite un convoyeur à vis installé dans une enveloppe, pouvant être chauffé à l'huile. Il sert à évacuer les déchets. Le réceptacle d'alimentation est isolé thermiquement et est doté d'un dispositif de production et d'injection de vapeur d'eau (20 à 31). L'extrémité de déchargement du convoyeur à vis 7 est pourvue, sur l'enveloppe, d'un canal d'éjection de l'air contaminé (12), dans lequel un ventilateur à aspiration (15) et un filtre (14) sont installés. L'installation est caractérisée par le fait que le réceptacle d'alimentation (1) peut être entièrement recouvert par un conteneur à déchets (18) en position retournée dont l'intérieur peut être désinfecté par de la vapeur d'eau projetée par un tuyau d'injection (31).

2. Installation de désinfection selon la revendication 1, caractérisée par le fait que le tube de projection de vapeur (31) est pivotant.

3. Installation de désinfection selon la revendication 1, avec un convoyeur à vis, composé de deux vis sans fin en mouvement de rotation contraire, caractérisé par le fait que les spires des deux vis sans fin (7a) sont différemment inclinées, de telle sorte qu'en raison du mouvement de rotation inverse de celles-ci, un tronçon donné de la vis à faible inclinaison fera face à un tronçon plus fortement incliné de la vis voisine.

4. Installation de désinfection selon la revendication 1, caractérisée par le fait qu'elle comprend un échangeur de chaleur (24) dont le circuit primaire est parcouru par l'huile refroidie et dont le circuit secondaire est parcouru par l'eau provenant du générateur de vapeur (22).

5. Installation de désinfection selon la revendication 1, caractérisée par le fait que la commande d'ouverture et de fermeture du couvercle (17) n'est conjuguée à une commande d'injection de la vapeur d'eau dans le réceptacle d'alimentation que lorsque le couvercle est ouvert.

6. Installation de désinfection selon la revendication 1, caractérisée par le fait que dans la partie supérieure en forme de trémie (2) du réceptacle d'alimentation (1) se trouve un rouleau vertical rotatif (19) monté sur excentrique et/ou dont la section n'est pas circulaire.

7. Installation de désinfection selon la revendication 1, caractérisée par le fait que la section libre du réceptacle d'alimentation (1) est évasée en-dessous du dispositif de déchiquetage (3, 4).

8. Installation de désinfection selon les revendications 1 et 6, caractérisée par le fait qu'un second dispositif de déchiquetage (4) est prévu.

9. Installation de désinfection selon au moins l'une des revendications 1 à 7, caractérisée par le fait que la plate-forme mobile (45), par exemple un genre de semi-remorque, accueille l'ensemble de l'installation de désinfection, y compris un groupe d'alimentation (48) pour le transport de gaz et chauffage et d'eau pour la production de vapeur, d'une grue hydraulique (56) pour la manipulation de petits conteneurs de déchets (18), dans la zone d'évolution de laquelle se trouve un dispositif de désinfection des conteneurs vides, des emplacements de rangement (55) pour un grand nombre de conteneurs étant prévus au moins de part et d'autre du convoyeur à vis chauffé (7). Ces petits conteneurs ont une capacité suffisante pour permettre le transport par la route des matières contaminées, la hauteur totale de la plate-forme mobile, superstructures comprises, ne devant pas dépasser la hauteur admissible pour la circulation routière.

10. Installation de désinfection selon la revendication 8, caractérisée par le fait que la partie supérieure (8) du réceptacle d'alimentation vertical (1) est démontable ou basculable.
